# EUROPÄISCHE PATENTSCHRIFT

(11) **EP 1 267 966 B1**
(45) Veröffentlichungstag und Bekanntmachung des Hinweises auf die Patenterteilung: **09.11.2005**
(21) Anmeldenummer: 01903556.7
(22) Anmeldetag: 16.02.2001
(51) Int. Cl.: A61M 5/32, A61M 5/50

(54) **EINWEG-INJEKTORKAPPE**
DISPOSABLE INJECTOR CAP
CAPUCHON D'INJECTEUR JETABLE

(30) Priorität: 01.03.2000 DE 10009814
(43) Veröffentlichungstag der Anmeldung: 02.01.2003
(73) Patentinhaber: TecPharma Licensing AG, 3401 Burgdorf (CH)
(72) Erfinder: HEINIGER, Hanspeter, CH-4932 Lotzwil (CH)
(86) Internationale Anmeldenummer: PCT/CH2001/000104
(87) Internationale Veröffentlichungsnummer: WO 2001/064270

(56) Entgegenhaltungen:
- DE-U- 29 820 166
- US-A- 5 405 362
- US-A- 5 429 612
- US-A- 5 681 291
- US-A- 5 688 241

## Beschreibung

Die Erfindung betrifft eine Injektorkappe für einen Ampullenträger. Insbesondere bezieht sich die Erfindung auf das Gebiet der sogenannten Pens, nämlich solcher Injektoren, die zur hypodermischen Verabreichung von Medikamenten verwendet werden, beispielsweise zur Verabreichung von Insulin für Diabetiker. Eine Injektorkappe gemäß dem Oberbegriff des Anspruchs 1 ist aus US-A-5 405 362 bekannt.

Die einfache Ausführungsform solcher Pens weist einen Ampullenträger auf, an dem an einer Stirnseite ein Kanülenträger mit einer Kanüle angeordnet ist. Über der Kanüle bzw. Nadel ist eine Kappe befestigt, die im Grundzustand die Kanüle bzw. Nadel völlig verdeckt. Die Kappe kann von dieser Position aus in Richtung des Ampullenträgers zurückgeschoben werden, wobei die Kanüle aus einem an der vorderen Stirnseite der Kappe ausgebildeten Loch austritt. Es ist deshalb möglich, den Pen an der Haut anzusetzen, anzudrücken, so dass die Kappe sich nach hinten gegen eine Federkraft verschiebt, die Injektion zu verabreichen und den Pen wieder abzuziehen, wobei die Federkraft dafür sorgt, dass die Kappe wieder in ihre Ausgangsstellung zurückgeschoben wird und die Nadel wiederum verdeckt. So kann sich der Verwender eine Injektion verabreichen, ohne die Kanüle auch nur einmal zu sehen, was insbesondere dann von Vorteil ist, wenn Patienten, die Ängste oder Abneigungen gegenüber solchen Nadeln haben, auf diese Behandlungen angewiesen sind. Außerdem schützt die Kappe die Kanüle vor Verunreinigungen.

Aus der US-A 5,609,577 ist ein "Pen" bekannt, bei dem die Kappe erst nach dem Aufdrehen eines Haltemechanismus gegen eine Federkraft zurückgeschoben werden kann, dies jedoch auch immer wieder} nach der Erstverwendung.

Ein Nachteil solcher herkömmlichen Injektoren besteht darin, dass nach der Verwendung die Kanüle ohne weiteres wieder freigelegt werden kann. Hierdurch besteht grundsätzlich die Gefahr, dass eine nicht mehr sterile Kanüle ein zweites Mal verwendet wird und dabei Infektionen verursacht. Ferner kann man sich an einer solch freigelegten Kanüle verletzen und auch dabei können Krankheiten übertragen werden, was im Zeitalter von Aids sogar lebensgefährlich sein kann.

Es ist deshalb die Aufgabe der vorliegenden Erfindung, eine Injektorkappe zur Verfügung zu stellen, welche die obigen Nachteile nicht mehr aufweist. Insbesondere soll in einfacher aber sicherer Weise verhindert werden, dass die Kanüle eines mit der Kappe versehenen Injektors nach der einmaligen bestimmungsgemäßen Verwendung nochmals benutzt oder freigelegt werden kann.

Diese Aufgabe wird erfindungsgemäß durch eine Injektorkappe für einen Ampullenträger gelöst, die eine auf diesen verschieblich gelagerte Schiebehülse aufweist, wobei die Schiebehülse über eine Vorspanneinrichtung in eine erste Position gedrückt wird, in der sie eine Kanüle verdeckt, und wobei die Schiebehülse gegen die Vorspannung in eine zweite Position verschoben werden kann, in der die Kanüle aus dem vorderen Stirnende der Schiebehülse austritt, wobei eine Sperreinrichtung vorgesehen ist, welche die Schiebehülse unlösbar gegen eine weitere Verschiebung sperrt, wenn sie von der zweiten Position wieder in die erste Position zurücküberführt worden ist.

Der Vorteil der erfindungsgemäßen Injektorkappe liegt darin, dass ein mit dieser ausgestatteter Injektor nach dem einmaligen bestimmungsgemäßen Gebrauch nicht nochmals benutzt werden kann. Ferner ist es nicht mehr möglich die schon einmal benutzte Kanüle nochmals freizulegen, so dass die Verletzungsgefahr und damit auch die Infektionsgefahr praktisch vollständig ausgeräumt werden.

Bei einer bevorzugten Ausführungsform weist die Sperreinrichtung Rastmittel auf, mittels der die Schiebehülse gegenüber einem feststehenden Teil des Ampullenträgers nach der Rückkehr in der ersten Position verrastet wird. Insbesondere kann die Sperreinrichtung einen Rastring aufweisen, der in der Schiebehülse verschieblich gelagert ist und beim Verschieben der Schiebehülse in die zweite Position hinter einem Rastvorsprung eines am Ampullenträger befestigten Kanülenträgers einrastet.

Bei einer Ausführung der zuletzt genannten Variante weist der Rastring umfänglich angeordnete und nach innen zusammenlaufende Rastlaschen auf, die hinter dem Rastvorsprung einrasten.

Es besteht die Möglichkeit, an dem Rastring zusätzlich Abstandslaschen vorzusehen, gegen welche die Schiebehülse stößt. Dieser Anstoß erfolgt nach der Rückkehr der Schiebehülse in die erste Position, und er wird vorzugsweise über federnde Stege an der Schiebehülse bewirkt.

Gemäß einer bevorzugten Ausführungsform der letztgenannten Variante weist die Schiebehülse einen Anschlag für die Abstandslaschen des Rastrings auf, der beim Überführen der Schiebehülse von der ersten in die zweite Position den Rastring über den Kanülenträger schiebt, bis die Rastlaschen hinter dem Rastvorsprung des Kanülenträgers einrasten.

Die Erfindung betrifft ferner einen Injektor mit einem Ampullenträger und einer Injektorkappe, die gemäß einer oder mehrerer vorstehend beschriebenen Ausführungsformen ausgestaltet ist. In bevorzugter Ausführung ist hierbei die Injektorkappe bzw. der Kanülenträger über eine Gewindeeinrichtung auf dem Ampullenträger aufgebracht. Wenn diese Komponenten aufgeschraubt werden, besteht nicht mehr die Gefahr, dass die Injektorkappe schon beim erstmaligen Anbringen an dem Ampullenträger durch den Sperrmechanismus arretiert und damit schon eine bestimmungsgemäße Verwendung verhindert wird.

Die Erfindung wird im weiteren Anhang einer bevorzugten Ausführungsform mittels der beiliegenden Zeichnungen näher erläutert. Es zeigen:
- Figur 1: eine perspektivische Darstellung einer aufgeschnittenen erfindungsgemäßen Injektorkappe an einem Ampullenträger im Ausgangszustand;
- Figur 2: einen Längsschnitt durch die in Figur 1 gezeigte Anordnung im Ausgangszustand;
- Figur 3: einen Längsschnitt durch die die erfindungsgemäße Injektoranordnung mit der Injektorkappe in vollständig zurückgeschobenem Zustand (zweite Position); und
- Figur 4: eine erfindungsgemäße Injektoranordnung im Längsschnitt nach dem bestimmungsgemäßen Gebrauch, wobei die Injektorkappe in den Ausgangszustand zurückverschoben und gegen eine weitere Verschiebung gesperrt ist.

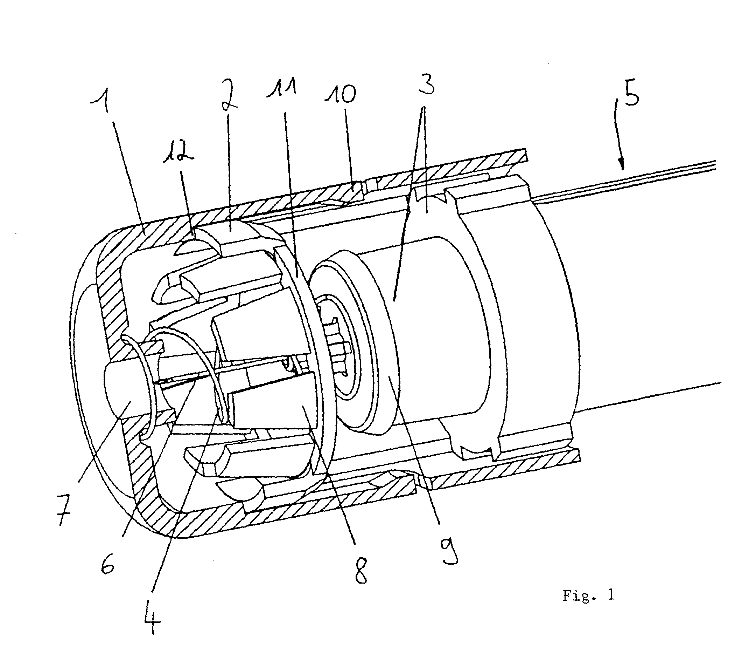

Anhand der Figur 1 wird nun zunächst der Aufbau einer erfindungsgemäßen Injektorkappe und deren Anbringung an einem Ampullenträger erläutert.

In den Figuren ist der Ampullenträger mit dem Bezugszeichen 5 gekennzeichnet. An seinem vorderen Stirnende ist ein Kanülenträger 3 aufgesetzt, der wiederum stirnseitig und mittig die Kanüle 6 nach vorne vorstehend hält. Der Kanülenträger 3 weist an dem Vorderende seines rundzylindrischen Abschnittes einen kreisförmig verlaufenden Rastvorsprung 9 auf, der an seinem den Ampullenträger 5 zugewandten Ende einen Absatz bildet und zur anderen Seite verjüngt zuläuft.

Die oben beschriebenen Bauteile sind diejenigen, die in ihrer Position fixiert sind.

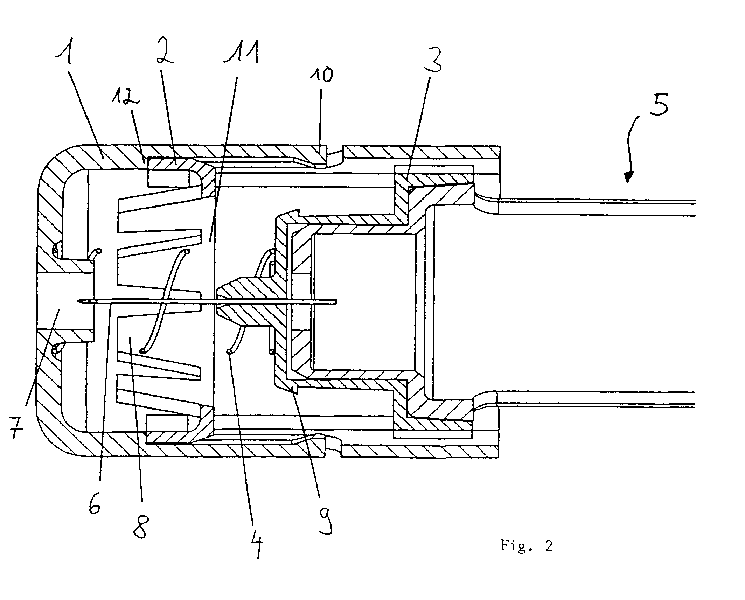

Kappenartig und verschieblich auf dem Ampullenträger 5 gelagert ist in Figur 1 und 2 die aufgeschnittene Schiebehülse 1 dargestellt. Ebenso wie die im weiteren beschriebenen Bauteile gehört sie zu den beweglichen Komponenten der erfindungsgemäßen Injektorkappe.

In dem in Figur 1 und 2 dargestellten Ausgangszustand ist der Injektor zur Verabreichung einer Injektion bereit. Hierzu befindet sich die Schiebehülse 1 in einer ersten Position, in der ihre Stirnseite den größten Abstand von dem Ampullenträger 5 aufweist. Die Kanüle 4 ist in diesem Zustand vollständig innerhalb der Schiebehülse 1 verborgen. Die Schiebehülse 1 wird durch eine Feder 4 zwischen dem Stirnende des Kanülenträgers 3 und einen nicht bezeichneten, inneren Ansatz am Stirnende der Schiebehülse 1 in der Position gehalten, in der sie in den Figuren 1 und 2 dargestellt ist. An der vorderen Stirnwand der Schiebehülse 1 ist eine kreisrunde Öffnung 7 vorgesehen, um eine Austrittsmöglichkeit für die Kanüle 7 zur Verfügung zu stellen.

Ein Rastring, der insgesamt mit 11 bezeichnet ist, ist in einer Führung verschieblich innen in der Schiebehülse 1 gelagert. In dem Ausgangszustand, der in den Figuren 1 und 2 dargestellt ist, weist der Rastring 11 innere, umfänglich angeordnete und nach vorne und innen zusammenlaufende Rastlaschen 8 auf, sowie zwei weiter außen und ebenfalls nach vorne abstehende Abstandslaschen 2. Mit den Abstandslaschen 2, d.h. mit deren vorderen Stirnkante schlägt der Rastring 11 an dem Anschlag 12 der Schiebehülse an. Der Anschlag 12 bildet das vordere Ende der Führung für den Rastring 11, die über eine bestimmte Länge aus dem inneren der Schiebehülse herausgearbeitet ist.

In diesem Bereich weist die Schiebehülse auch zwei einander gegenüberliegende aus dem umgebenden Material freigelegte Stege 10 auf, die an ihrem freien Ende einen schräg nach innen vorstehenden Absatz ausbilden. Die Stege 10 können in Radialrichtung elastisch verformt werden.

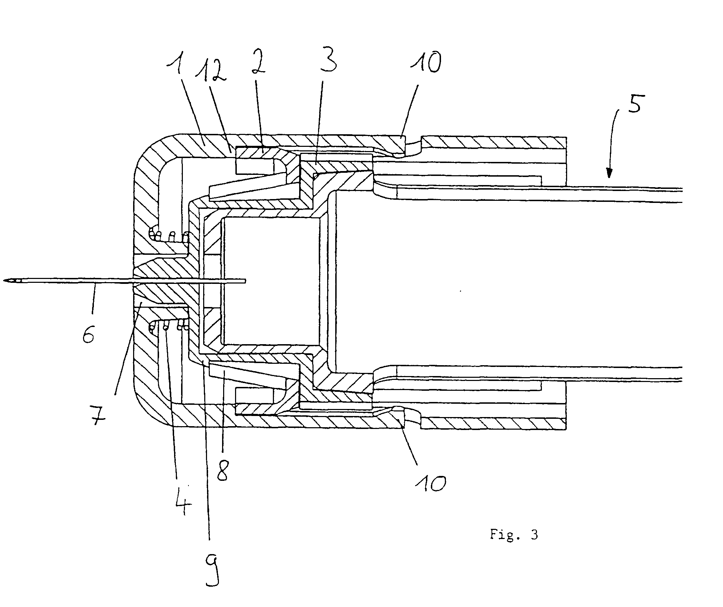

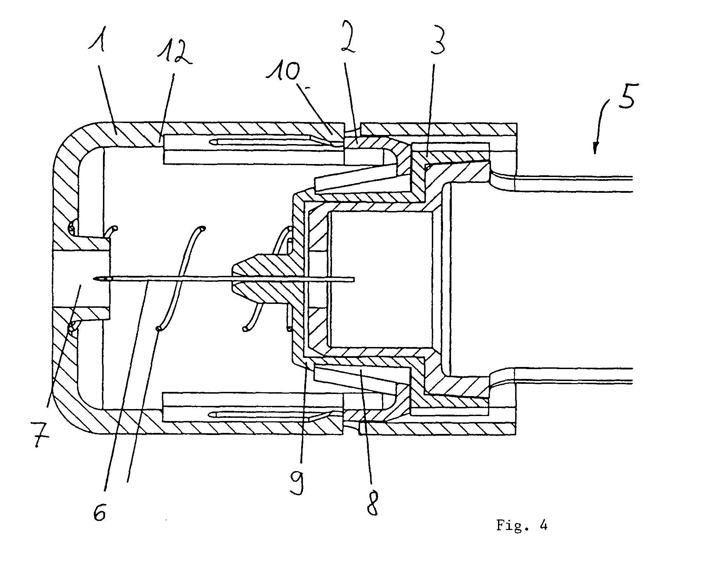

Ausgehend von dem Zustand, der in Figur 1 und 2 dargestellt ist, kann nunmehr anhand der Darstellungen der Figuren 3 und 4 aufgezeigt werden, wie die erfindungsgemäße Injektorkappe funktioniert.

Wenn mittels des Injektors eine Medikamentendosis verabreicht werden soll, so wird dieser mit der vorderen Stirnseite der Schiebehülse 1 an der Haut eines Patienten angesetzt.

Danach wird der Ampullenträger 5 nach vorne angeschoben, so dass die Schiebehülse 1 sich relativ zum Ampullenträger 5 nach hinten schiebt und zwar gegen die Kraft der Feder 4 und solange, bis der Kanülenträger 3 mit seinem vorderen Stirnende an dem inneren Ansatz anschlägt, der die Öffnung 7 umgibt und vorne von der Feder 4 umschlossen wird. Dieser Zustand ist in Figur 3 gezeigt.

Durch die vorher beschriebene Betätigung ändert sich an der relativen Lage des Rastringes 11 mit den Laschen 8 und 2 gegenüber der Schiebehülse 1 nichts, d.h. die Abstandslaschen 2 drücken noch immer gegen den Anschlag 12. Was sich jedoch ändert, ist die relative Lage des Rastringes 11 gegenüber dem Kanülenträger 3; der Kanülenträger 3 wird nämlich zusammen mit dem Ampullenträger 5 nach vorne in den Rastring hinein durch die Rastlaschen 8 hindurchgeschoben. Hierbei werden die Rastlaschen 8 beim Durchgang des Rastvorsprunges 9 zunächst etwas elastisch nach außen gedrückt und schnappen dann hinter dem Rastvorsprung 9 ein, wodurch der Rastring 11 gegenüber dem Kanülenträger 3 fixiert wird.

In diesem Zustand ist die Kanüle 6 weit aus der Öffnung 7 ausgefahren und die Injektion kann verabreicht werden.

Nach dem Verabreichen der Injektion bewirkt die Feder 4, das die Schiebehülse 1 wieder von dem Kanülenträger 3 weggeschoben wird und es wird ein Zustand erreicht, wie er in Figur 4 dargestellt ist.

Da, wie in Figur 3 dargestellt ist und oben schon erwähnt wurde, der Rastring 11 nunmehr durch die Rastlaschen 8 hinter dem Rastvorsprung 9 an dem Kanülenträger 3 fixiert ist, bleibt er beim Wieder-nach-vorne-Bringen der Schiebehülse 1 auch fixiert, d.h. die Schiebehülse 1 geht wieder nach vorne, ohne den Rastring mitzunehmen. Bei dieser Bewegung der Schiebehülse 1 nach vorne gleiten nunmehr die Stege 10 an dem Außenumfang der Abstandslaschen 2 entlang und werden kurzzeitig durch das Überlaufen des hinteren Absatzes über die Abstandslaschen 2 elastisch nach außen gedrückt, bevor sie mit ihrem hinteren Stirnende über das vordere Ende der Abstandslaschen 2 hinweg nach innen zurückschnappen. Nach diesem Zurückschnappen sind die Stege 10 in der Position, wie sie in Figur 4 dargestellt ist, d.h. sie stoßen stirnseitig an die vordere Fläche der Abstandslaschen 2. Dieser Anstoß verhindert ein nochmaliges Zurückschieben der Schiebehülse 1 gegenüber dem Kanülenträger 3 bzw, dem Ampullenträger 5.

In dem Zustand, der sich gemäß Figur 4 nach der einmaligen Verabreichung der Medikamentendosis einstellt, ist damit die Schiebehülse 1 gegen eine weitere Verschiebung gesperrt und deckt die Kanüle 6 wieder vollständig gegen die Umgebung ab. Damit wird eine nochmalige Verwendung des Injektors positiv verhindert. Ebenso wird verhindert, dass man sich an der Nadel verletzen oder infizieren kann.

Die Rast- bzw. Sperreinrichtungen einer erfindungsgemäßen Injektorkappe befinden sich im wesentlichen alle im Inneren der Kappe oder sind mit ihren Funktionsteilen dem Inneren zugewandt, so dass eine Wiederverwendung, die nicht bestimmungsgemäß geschehen soll, also beispielsweise eine zweite Verwendung, nur durch weitgehende Manipulationen bewirkbar ist, welche wohl zur Zerstörung des Injektors bzw. der Injektorkappe führen würden.

## Patentansprüche

1. Injektorkappe für einen Ampullenträger (5) mit einer auf diesem verschieblich gelagerten Schiebehülse (1), wobei die Schiebehülse (1) über eine Vorspanneinrichtung (4) in eine erste Position gedrängt wird, in der sie eine Kanüle (6) verdeckt, und wobei die Schiebehülse (1) gegen die Vorspannung in eine zweite Position verschoben werden kann, in der die Kanüle (6) aus dem vorderen Stirnende der Schiebehülse (1) austritt, und mit einer Sperreinrichtung (9, 10, 11), welche die Schiebehülse (1) unlösbar gegen eine weitere Verschiebung sperrt, wenn sie von der zweiten Position wieder in die erste Position zurück überführt worden ist, wobei die Sperreinrichtung wenigstens ein Rastmittel (11) aufweist, **dadurch gekennzeichnet, daß** das Rastmittel (11) in der Schiebehülse (1) verschieblich gelagert ist.

2. Injektorkappe nach Anspruch 1, **dadurch gekennzeichnet, dass** das wenigstens eine Rastmittel (11) beim Verschieben in die zweite Position relativ zu der Schiebehülse (1) in Ruhe ist und beim Verschieben zurück in die erste Position relativ zu dem Ampullenträger (5) in Ruhe ist.

3. Injektorkappe nach Anspruch 1 oder 2, **dadurch gekennzeichnet, dass** die Sperreinrichtung (9, 10, 11) die Schiebehülse (1) gegenüber einem feststehenden Teil des Ampullenträgers (5) nach der Rückkehr in der ersten Position verrastet wird.

4. Injektorkappe nach einem der Ansprüche 1 bis 3, **dadurch gekennzeichnet, dass** das wenigstens eine Rastmittel (11) von einem Rastring gebildet wird, der beim Verschieben der Schiebehülse (1) in die zweite Position hinter einem Rastvorsprung (9) eines am Ampullenträger (5) befestigten Kanülenträgers (3) einrastet.

5. Injektorkappe nach einem der vorhergehenden Ansprüche, **dadurch gekennzeichnet, dass** der Rastring (11) umfänglich angeordnete und nach innen zusammenlaufende Rastlaschen (8) aufweist, die hinter dem Rastvorsprung (9) einrasten.

6. Injektorkappe nach einem der vorhergehenden Ansprüche, **dadurch gekennzeichnet, dass** der Rastring (11) Abstandslaschen (2) aufweist gegen welche die Schiebehülse (1), vorzugsweise über federnde Stege (10), nach der Rückkehr in die erste Position stösst.

7. Injektorkappe nach Anspruch 6, **dadurch gekennzeichnet, dass** die Schiebehülse (1) einen Anschlag (12) für die Abstandslaschen (2) des Rastrings (11) aufweist, der beim Überführen der Schiebehülse (1) von der ersten in die zweite Position den Rastring (12) über den Kanülenträger (3) schiebt, bis die Rastlaschen (8) hinter dem Rastvorsprung (9) einrasten.

8. Injektor mit einem Ampullenträger (5) und einer Injektorkappe nach einem der Ansprüche 1 bis 7.

9. Injektor nach Anspruch 8, **dadurch gekennzeichnet, dass** die Injektorkappe bzw. der Kanülenträger (3) über eine Gewindeeinrichtung auf dem Ampullenträger (5) aufgebracht ist.

## Claims

1. An injector cap for an ampoule carrier (5) comprising a sliding sleeve (1) mounted slidably on the ampoule carrier, wherein the sliding sleeve (1) is urged by way of a biasing device (4) into a first position in which it covers a cannula (6) and wherein the sliding sleeve (1) can be slid against the bias into a second position in which the cannula (6) issues from the front end of the sliding sleeve (1), and a locking device (9, 10, 11) which locks the sliding sleeve (1) non-releasably to prevent further sliding movement when it has been moved from the second position back into the first position again, wherein the locking device has at least one latching means (11) **characterised in that** the latching means (11) is mounted slidably in the sliding sleeve (1).

2. An injector cap according to claim 1 **characterised in that** the at least one latching means (11) is at rest upon displacement into the second position relative to the sliding sleeve (1) and is at rest upon displacement back into the first position relative to the ampoule carrier (5).

3. An injector cap according to claim 1 or claim 2 **characterised in that** the locking device (9, 10, 11) latches the sliding sleeve (1) with respect to a fixed part of the ampoule carrier (5) after return in the first position.

4. An injector cap according to one of claims 1 to 3 **characterised in that** the at least one latching means (11) is formed by a latching ring which upon displacement of the sliding sleeve (1) into the second position latches behind a latching projection (9) of a cannula carrier (3) fixed to the ampoule carrier (5).

5. An injector cap according to one of the preceding claims **characterised in that** the latching ring (11) has peripherally arranged and inwardly converging latching tongues (8) which latch behind the latching projection (9).

6. An injector cap according to one of the preceding claims **characterised in that** the latching ring (11) has spacing tongues (2) against which the sliding sleeve (1) butts, preferably by way of resilient legs (10), after the return into the first position.

7. An injector cap according to claim 6 **characterised in that** the sliding sleeve (1) has an abutment (12) for the spacing tongues (2) of the latching ring (11), which, when the sliding sleeve (1) is transferred from the first position into the second position, pushes the latching ring (12) over the cannula carrier (3) until the latching tongues (8) latch behind the latching projection (9).

8. An injector having an ampoule carrier (5) and an injector cap according to one of claims 1 to 7.

9. An injector according to claim 8 **characterised in that** the injector cap or the cannula carrier (3) is mounted on the ampoule carrier (5) by way of a screwthread means.

## Revendications

1. Capuchon d'injecteur pour un support d'ampoule (5), comportant une douille coulissante (1) monté de manière à être déplaçable sur ce support d'ampoule, dans lequel la douille coulissante (1) est repoussée au moyen d'un dispositif de précontrainte (4) dans une première position, dans laquelle elle recouvre une canule (6), et dans lequel la douille coulissante (1) peut être déplacée à l'encontre de la précontrainte pour venir dans une seconde position, dans laquelle la canule (6) ressort de l'extrémité frontale avant de la douille coulissante (1), et comportant un dispositif de blocage (9, 10, 11), qui bloque d'une manière inamovible la douille coulissante (1) contre un déplacement supplémentaire, lorsqu'elle a été ramenée à nouveau de la seconde position dans la première position, le dispositif de blocage comportant au moins un moyen d'encliquetage (11), **caractérisé en ce que** le moyen d'encliquetage (11) est monté de manière à être déplaçable dans la douille coulissante (1).

2. Capuchon d'injecteur selon la revendication 1, **caractérisé en ce que** le au moins un moyen d'encliquetage (11) est au repos lors du déplacement aboutissant à la seconde position par rapport à la douille coulissante (1) et est au repos lors du déplacement en retour dans la première position par rapport au support d'ampoule (5).

3. Capuchon d'injecteur selon la revendication 1 ou 2, **caractérisé en ce que** le dispositif de blocage (9, 10, 11) réalise l'encliquetage de la douille coulissante (1) par rapport à une partie fixe du support d'ampoule (5), après le retour dans la première position.

4. Capuchon d'injecteur selon l'une des revendications 1 à 3, **caractérisé en ce que** le au moins un moyen d'encliquetage (11) est formé par une bague d'encliquetage qui, lors du déplacement de la douille coulissante (1) venant dans la seconde position, s'encliquette derrière une partie saillante d'encliquetage (9) d'un porte-canule (3) fixé sur le support d'ampoule (5) .

5. Capuchon d'injecteur selon l'une des revendications précédentes, **caractérisé en ce que** la bague d'encliquetage (11) possède des pattes d'encliquetage (8) qui sont disposées circonférentiellement, convergent vers l'intérieur et s'encliquettent derrière la partie saillante d'encliquetage (9).

6. Capuchon d'injecteur selon l'une des revendications précédentes, **caractérisé en ce que** la bague d'encliquetage (11) comporte des pattes d'entretoisement (2), contre lesquelles la douille coulissante (1) s'applique après le retour dans la première position, de préférence au moyen de barrettes élastiques (10).

7. Capuchon d'injecteur selon la revendication 6, **caractérisé en ce que** la douille coulissante (1) possède une butée (12) prévue pour les pattes d'entretoisement (2) de la bague d'encliquetage (11) et qui, lors du passage de la douille coulissante (1) de la première position à la seconde position, déplace la bague d'encliquetage (12) au-dessus du porte-canule (3), jusqu'à ce que les pattes d'encliquetage (6) s'encliquètent derrière la partie saillante d'encliquetage (9).

8. Injecteur comportant un support d'ampoule (5) et un capuchon d'injecteur selon l'une des revendications 1 à 7.

9. Injecteur selon la revendication 8,
**caractérisé en ce que** le capuchon d'injecteur ou le support de canule (3) est monté au moyen d'un dispositif fileté sur le support d'ampoule (5).
